# EUROPEAN PATENT APPLICATION

(11) **EP 3 338 805 A1**
(43) Date of publication of application: **27.06.2018**
(21) Application number: 16814530.8
(22) Date of filing: 25.06.2016
(51) Int. Cl.: A61K 47/42, A61P 35/00, C07K 2/00, C07K 14/00

(54) **DRUG COMPLEX**

(30) Priority: 25.06.2015 JP 2015128028
(71) Applicant: Shimadzu Corporation, Kyoto 604-8511 (JP)
(72) Inventor: FUJII, Ikuo, Sakai-shi Osaka 599-8531 (JP)
(74) Representative: Webster, Jeremy Mark
(86) International application number: PCT/JP2016/068934
(87) International publication number: WO 2016/208761

(57) **Abstract**

[Problem to be solved] To provide a drug delivery system capable of delivering a drug into a cell expressing a VEGF receptor.

[Solution] Provided is a VEGF-binding peptide-drug complex in which a drug is bound to a C-terminal amino acid /or an N-terminal amino acid of a VEGF-binding peptide and is incorporated into a VEGF receptor-expressing cell by endocytosis of a VEGF receptor, the VEGF-binding peptide having a helix-loop-helix structure including an A block that includes a peptide forming an α-helix structure and is positioned on an N-terminal side, a C block that includes a peptide forming an α-helix structure and is positioned on a C-terminal side, and a B block that includes a peptide linking the A block and the C block by a covalent bond.

## Description

### [Technical Field]

The present invention relates to a drug complex, more specifically to a peptide-drug complex in which a drug is bound to a VGEF-binding peptide.

### [Technical Background]

A vascular endothelial growth factor (VEGF) is generally known as a protein that promotes angiogenesis. This VEGF plays an important role in formation of cardiovascular system and construction of many tissues in vertebrate fetal period and early life. However, since VEGF is involved in growth and metastasis of cancer, pathogenesis and promotion of rheumatoid arthritis, diabetic retinopathy and the like after maturity, VEGF is regarded as important to such pathological conditions.

VEGF exerts a biological effect by binding to or interacting with a transmembrane tyrosine kinase receptor VEGFR. Therefore, by inhibiting binding or interaction between VEGF and VEGFR, angiogenesis is suppressed, and this is expected to lead to suppression of cancer growth and metastasis, and prevention of rheumatoid arthritis and diabetic retinopathy, prevention of accelerated pathology of age-related macular degeneration, and the like. In fact, bevacizumab (trade name, Avastin), which is a monoclonal antibody that binds to VEGFR and thereby inhibits the binding or interaction with VEGF, is commercially available and is used as an anti-cancer agent for metastatic colorectal cancer and metastatic breast cancer.

However, when an antagonist is a peptide, in general, a peptide of which a steric structure is restricted is selected. For a peptide having many restricted steric structures, the steric structures are stabilized by disulfide bonds in the peptide. However, there is a disadvantage that the disulfide bonds are cleaved under an Intracellular reduction condition and the steric structures are easily broken. Further, there is also a disadvantage that decomposition occurs due to in vivo protease and a half-life in serum is short. Therefore, a more stable peptide in vivo is required.

As a stabilized peptide that does not have such disadvantages, a peptide having a helix-loop-helix structure is disclosed in Patent Document 1 and the like. A peptide having a helix-loop-helix structure has an N-terminal side amino acid sequence (N-terminal side helix: A block), a C-terminal side amino acid sequence (C-terminal side helix: C block), and a linker (B block) that binds the A block and the C block to each other. The A block and the C block respectively form α-helical coiled-coil structures due to the presence of the linker. Although having a low molecular structure, this peptide has a stable secondary structure in a solution, and a functional group having a chemically different property can be easily introduced to a portion exposed on a solvent side in a molecule. Using such a property, various peptides having a helix-loop-helix structure having physiological activity have been proposed.

Under such circumstances, the present inventors have proposed a VEGF-binding peptide complex that has a VEGF-binding property and inhibits binding between a VEGF receptor and a VEGF (Patent Document 2). This complex is a complex in which thioredoxin is bound to a C-terminal side of a peptide having a helix-loop-helix structure having a VEGF-binding property. A VEGF-binding peptide does not inhibit interaction between VEGF and VEGF receptor, or its inhibitory ability is extremely small, and it is considered that a VEGF-binding peptide alone cannot achieve a sufficient anti-cancer effect. Therefore, a purpose is to inhibit binding or interaction between a VEGF and a VEGF receptor, suppress angiogenesis, and achieve an anti-cancer effect and the like by a steric hindrance resulting from binding a molecule having a large steric structure such as thioredoxin to a VEGF-binding peptide. However, binding inhibitory activity between the two in this complex is still insufficient. Further, there is also a problem that, when a molecule of the complex becomes large, a binding property of the VEGF-binding peptide itself is weakened or production thereof becomes difficult, and a new cancer therapeutic agent that overcomes these problems is demanded.

### [Related Art]

### [Patent Documents]

[Patent Document 1] Japanese Patent Laid-Open Publication No. HEI 10-245397.
[Patent Document 2] Japanese Patent Laid-Open Publication No. 2014-245397.

### [Summary of the Invention]

### [Problems to Be Solved by the Invention]

A problem to be solved by the present invention is to provide a drug delivery system capable of specifically delivering a drug showing cytotoxicity into a cell expressing a VEGF receptor, in particular, a cancer cell, without affecting a normal cell.

### [Means for Solving the Problems]

The present inventors accomplished the present invention by utilizing that a VEGF receptor expressed on a cell surface performs endocytosis that, when a VEGF binds to an extracellular binding site, the conjugate is incorporated into the cell, and by focusing on that the above-described VEGF-binding peptide is also similarly incorporated into a cell. That is, in the present invention, a drug complex is provided in which a drug is bound to a peptide having a helix-loop-helix structure having a VEGF-binding property, and the drug is incorporated into a cell expressing a VEGF receptor.

### [Effect of the Invention]

According to the present invention, a drug showing cytotoxicity can be specifically delivered to a cell expressing VEGF receptor, in particular, to a cancer cell, and the cancer cell can be specifically damaged.

### [Brief Description of the Drawings]

[Fig. 1] Fig. 1 is a conceptual diagram of a drug complex according to the present invention.
[Fig. 2] Fig. 2 is a conceptual diagram illustrating an example of a method for synthesizing a VEGF-binding peptide M49K capable of binding a drug.
[Fig. 3] Fig. 3 shows measurement results of a secondary structure of the VEGF-binding peptide M49K to which a drug can bind; (a) shows a sensorgram with respect to VEGF of M49K (C1A) obtained using an SPR method; (b) shows a sensorgram of a cyclic M49; and (c) shows CD spectra of M49 and M49K (C1A).

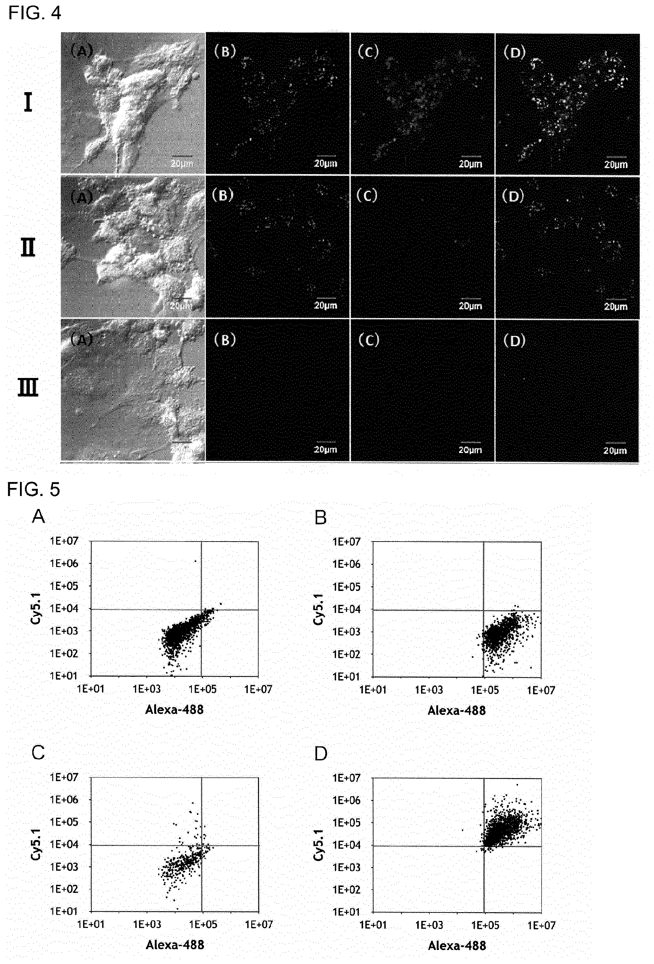

[Fig. 4] Fig. 4 shows confocal laser scanning microscope images showing intracellular incorporation of a VEGF-binding peptide M49K to which a drug can bind; (I) shows HUVEC to which Cy5-M49K and Alexa 488-VEGF were added; (II) shows HUVEC to which only Alexa 488-VEGF was added; (III) shows HUVEC to which only Cy5-M49K was added; and in each row, (A) shows a shape of cells captured using a phase contrast microscope; (B) shows a cell sectional view of Alexa 488 in which green fluorescence was observed; (C) shows a cell sectional view of red fluorescence of Cy5 was observed; and (D) shows a merge image of (B) and (C).
[Fig. 5] Fig. 5 shows results of a cell incorporation test of VEGF-binding peptide M49K, to which a drug can bind, using a flow cytometer. (A) shows untreated HUVEC; (B) shows HUVEC in which Alexa488-VEGF was processed; (C) shows HUVEC in which Cy5.1-M49K was processed; and (D) shows HUVEC in which Alexa488-VEGF and Cy5.1-M49K were processed.
[Fig. 6] Fig. 6 is a synthesis scheme of a drug complex which is an embodiment of the present invention.
[Fig. 7] Fig. 7 shows measurement results of binding activity of a drug complex M49K-Cem with respect to VEGF using an SPR method.
[Fig. 8] Fig. 8 shows results of a HUVEC growth inhibition test of a drug complex M49K-Cem; M49K shows VEGF-binding peptide M46K to which a drug as a control can bind; and YTI-Cem shows a non-VEGF-binding peptide-drug complex YT1-Cem as a control.

### [Mode for Carrying Out the Invention]

A drug complex according to the present invention is a VEGF-binding peptide-drug complex formed by binding a VEGF-binding peptide and a drug to each other, the VEGF-binding peptide having a helix-loop-helix structure including an A block that includes a peptide forming an α-helix structure and is positioned on an N-terminal side, a C block that includes a peptide forming an α-helix structure and is positioned on a C-terminal side, and a B block that includes a peptide linking the A block and the C block by a covalent bond. The drug directly or indirectly binds to an N-terminal amino acid and/or a C-terminal amino acid of the VEGF-binding peptide and is incorporated into a VEGF receptor-expressing cell by endocytosis of a VEGF receptor.

The VEGF-binding peptide used in the present invention is not particularly limited as long as the VEGF-binding peptide has the helix-loop-helix structure and has a VEGF-binding property. Since a VEGF-binding peptide is incorporated into a cell together with a bound VEGF, a VEGF-binding peptide having a strong VEGF-binding property is preferred. Specific examples of such a peptide having a strong VEGF-binding property are disclosed in Patent Document 2. Therefore, in the present specification, description of Patent Document 2 can be referred to as appropriate.

The helix-loop-helix structure is also referred to as an α-helical coiled-coil structure, and a peptide having this structure stably exists in a solution as a single molecule. This peptide is stabilized by a hydrophobic interaction of leucine arranged between the two helixes (the A block and the C block). Further, it is designed such that a salt bridge (the B block) is formed between a glutamic acid side chain of an N-terminal side α-helix (the A block) and a lysine side chain of a C-terminal side α-helix (the C block). This peptide retains a stable steric structure even when other residues are randomized as long as an amino acid residue important for the formation of these steric structures remains.

The VEGF-binding peptide according to the present invention is a peptide having such a basic structure, and has a binding property with respect to VEGF. Whether or not having a binding property with respect to VEGF can be determined based on a dissociation constant (K_{D}) with respect to VEGF obtained using a method described in Patent Document 2. For example, when the dissociation constant is 10,000 nM or less, the peptide can be determined as having a binding property with respect to VEGF. Further, in a case of a cyclic peptide formed by covalently bonding and cyclizing an amino acid at the N-terminal of the A block and an amino acid at the C-terminal of the C block, a dissociation constant (K_{D}) between the cyclic peptide and VEGF is 10,000 nM or less, preferably 1,000 nM or less, and more preferably 500 nM or less. More desirably, a VEGF-binding peptide having a dissociation constant (K_{D}) of 10 nM or less is preferred.

As a more specific sequence, in the present invention, a VEGF-binding peptide, in which amino acid sequences of the B block include amino acid sequences shown in SEQ ID NO: 3 (GTYRASTWWWG), SEQ ID NO: 4 (GPDLMVWWGWD), SEQ ID NO: 5 (GNSDYPWIGWG), and SEQ ID NO: 6 (GPWKGYPIPYG), is preferable. A peptide of a helix-loop-helix structure having a peptide, which includes amino acid sequences shown in SEQ ID NOs: 3 - 6, in the B block has a good VEGF-binding property.

An amino acid sequence of the A block or the C block is also not particularly limited as long as the amino acid sequence has the above basic structure. For example, a peptide of the A block can be a peptide including of an amino acid sequence shown in SEQ ID NO: 7 (CAAELAALEA ELAALE). Further, a peptide of the C block, for example, is a peptide including an amino acid sequence shown in SEQ ID NO: 8 (KLAALKAKLAALKAAC), is preferably a peptide obtained by substituting an amino acid (X), which is not essential for maintaining a steric structure among amino acid sequences shown in SEQ ID NO: 9 (peptide including an amino acid sequence shown in KLXXLKXKLXXLKXAC), with any amino acid other than threonine, alanine and proline, and is more preferably a peptide including amino acid sequences shown in SEQ ID NO: 10 (KLFQLKNKLHQLKYAC), SEQ ID NO: 11 (KLNQLKHKLDHLKVAC), SEQ ID NO: 12 (KLGELKQKLLKLKNAC), and SEQ ID NO: 13 (KLQFLIKKLKQLKVAC). In the present invention, an amino acid forming a peptide is a naturally occurring L-amino acid, and is particularly preferably an amino acid forming a protein, and may be a D-amino acid as long as a steric structure is maintained.

In the present invention, an amino acid sequence of the B block is important, and an amino acid sequence of the A block or an amino acid sequence of the C block can be arbitrary. Therefore, in the present invention, a C block including an amino acid sequence shown in any one of SEQ ID NOs: 10 - 13 may be combined with a B block including an amino acid sequence shown in any one of SEQ ID NOs: 3 - 6. More specifically, a VEGF-binding peptide having amino acid sequences shown in SEQ ID NOs: 14 - 17 is preferably used. Further, amino acid sequences obtained by deleting or substituting or inserting 1 or 2 amino acids in these amino acid sequences can also be used. Preferred amino acid sequences in the present invention are summarized in Table 1. Clones 31, 41, 42, 49 shown in Table 1 correspond to Clones 31, 41, 42, 49 described in Patent Document 2; M49 has the same amino acid sequence as Clone 49; and M49K is based on M49 and is a cyclic VEGF-binding peptide having a free condensable functional group in an N-terminal amino acid of the A block, the free condensable functional group being capable of binding a drug.

**[Table 1]**

| SEQ ID NO. | ABlock - B Block - C Block | |
|---|---|---|
| 1 | CAAELAALEAELAALEGPWKGYPIPYGKLQFLIKKLKQLKVAC | M49 |
| 2 | CAELAALEAELAALEGPWKGYPIPYGKLQFLIKKLKQLKVAGGGG | M49K |
| 3 | GTYRASTWWWG | Clone 36 |
| 4 | GPDLMVWWGWD | Clone 41 |
| 5 | GNSDYPWIGWG | Clone 42 |
| 6 | GPWKGYPIPYG | Clone 49 |
| 7 | CAAELAALEAELAALE | |
| 8 | KLAALKAKLAALKAAC | |
| 9 | KLXXLKXKLXXLKXAC | |
| 10 | KLFQLKNKLHQLKYAC | Clone 36 |
| 11 | KLNOLKHKLDHLKVAC | Clone 41 |
| 12 | KLGELKQKLLKLKNAC | Clone 42 |
| 13 | KLQFLIKKLKQLKVAC | Clone 49 |
| 14 | CAAELAALEAELAALEGTYRASTWWWGKLFQLKNKLHQLKYAC | Clone 36 |
| 15 | CAAELAALEAELAALEGPDLMVWWGWDKLNQLKHKLDHLKVAC | Clone 41 |
| 16 | CAAELAALEAELAALEGNSDYPWIGWGKLGELKQKLLKLKNAC | Clone 42 |
| 17 | CAAELAALEAELAALEGPWKGYPIPYGKLQFLIKKLKQLKVAC | Clone 49 |

The complex according to the present invention is a complex in which a drug is bound to a VEGF-binding peptide. A binding site of the drug is an N-terminal amino acid or a C-terminal amino acid of the VEGF-binding peptide, and is preferably the N-terminal amino acid.

A binding mode of the drug is not limited, and can be selected by taking into consideration a production process of the complex, an effect developing property of the drug, or the like. For example, in a case where the drug incorporated into a cell exerts a medicinal effect by detaching from the complex, a binding mode that allows easy detachment in a cell can be adopted. Further, from a point of view of not imparting toxicity to a normal cell, it is preferable to bind the drug in a binding mode in which detachment is difficult to occur in blood. A binding mode can use, for example, a condensable functional group of an N-terminal amino acid or a C-terminal amino acid. Here, the term "condensable functional group" means a functional group, such as a thiol group, a hydroxyl group, an amino group, a carboxyl group and an aldehyde group, capable of causing an addition-elimination reaction such as an esterification reaction (including a thioesterification reaction), an amidation reaction, and aldol condensation between the VEGF-binding peptide and the drug.

The condensable functional group may be a functional group of the N-terminal amino acid or the C-terminal amino acid of the VEGF-binding peptide itself, or may be an artificially introduced functional group. A functional group of an amino acid itself is, for example, a thiol group of cysteine, an α-position amino group of an amino acid at an N-terminal, a hydroxyl group of a serine group, a carboxyl group of an aspartic acid or glutamic acid at an N-terminal, or a carboxyl group of an amino acid at a C-terminal. In the present invention, for example, by allowing the VEGF-binding peptide having amino acid sequences shown in SEQ ID NOs: 14 - 17 to have such a condensable functional group, a drug can be bound. Such a VEGF-binding peptide can be easily obtained by inserting an amino acid having a functional group described above to an N-terminal or a C-terminal or by substituting an N-terminal amino acid or a C-terminal amino acid.

In the present invention, a VEGF-binding peptide having a cyclic structure in which an N-terminal amino acid and a C-terminal amino acid are directly or indirectly bonded to each other is preferably used. Even when a cyclic structure is not adopted, the VEGF-binding peptide is stable due to interaction between the A block and the C block. However, adopting a cyclic structure results in a more stable and potent VEGF-binding peptide. Here, the term "direct binding" means that the N-terminal amino acid of the A block and the C-terminal amino acid of the C block are bound to each other without intervention of a linker, for example, as in a case where the N-terminal amino acid of the A block and the C-terminal amino acid of the C block are bound to each other by an amide bond (peptide bond), or in a case where a N-terminal cysteine of the A block and a C-terminal cysteine of the C block are bound to each other by a disulfide bond (SS bond). The term "indirect binding" means that the N-terminal amino acid of the A block and the C-terminal amino acid of the C block are bound to each other via a linker. A structure of the linker is not particularly limited. Examples of the linker include a linker in which carbon atoms are linearly bonded, a linker in which an amino acid is peptide-bonded, and the like. A length of the linker is appropriately determined according to peptide stability, incorporation into a cell, an effect developing property of the drug, and the like. Examples include a linker in which 1 - 10 methylene groups are linearly bonded, and a peptide chain in which 1 - 9, preferably 1 - 5, and more preferably 1 - 4 amino acids are bonded by amide bonds. An amino acid forming the linker is not restricted. However, from a point of view of having a simple structure, glycine is preferable, and a peptide chain formed from glycine only is desirable as a linker. Further, a linker formed from a peptide chain does not adopt an α-helix structure, and a sequence that does not affect an α-helix structure of the A block or an α-helix structure of the C block is adopted.

Further, as long as stabilization of the helix-loop-helix structure is not hindered, the VEGF-binding peptide having the condensable functional group in the present invention can also use the amino acid sequence of the A block and/or the C block described above, or an amino acid sequence obtained by deleting or substituting or inserting 1 or 2 amino acids in an amino acid sequence of, for example, M49K having a linker.

The drug that binds to the VEGF-binding peptide is a drug that can be incorporated by endocytosis in a cell expressing a VEGF receptor. In the present invention, the term "a drug capable of being incorporated by endocytosis" means a drug that is incorporated into a cell without inhibiting binding of a VEGF and a VEGF receptor with a steric hindrance thereof when the drug is bound to a VEGF-binding peptide. A molecular weight of such a drug is approximately 30,000 or less, preferably 20,000 or less, more preferably 10,000 or less, and desirably 5,000 or less. An upper limit of the molecular weight is only a rough guide. In the present invention, bulkiness of the drug, that is, that the VEGF-binding peptide-drug complex is a molecule of a size that does not cause a steric hindrance is important. A preferred example of the drug is a toxic drug showing cytotoxicity when a cancer cell is assumed as a target tissue. Examples of the drug include doxorubicin hydrochloride, peplomycin hydrochloride, nitrogen mustard-N-oxide hydrochloride, cyclophosphamide, thiodepa, carboncon, nimustine hydrochloride, bleomycin hydrochloride, bleomycin sulfate, peplomycin sulfate, aclarubicin hydrochloride, idarubicin hydrochloride, epirubicin hydrochloride, daunorubicin hydrochloride, pirarubicin hydrochloride, ginostatin stimulamer, neocarcinostatin, etoposide, teniposide, irinotecan hydrochloride, vincristine sulfate, vindesine sulfate, vinblastine sulfate, L-asparaginase, mitoxantrone hydrochloride, cisplatin, carboplatin, nedaplatin, pentostatin, diszophylan, porphymer sodium, ifesfamide, catalabazine, mercaptopurine, thioinosine, cytarabine, enocitabine, fluorouracil, tegafur, ancitabine hydrochloride, methotrexate, carmofur, mitomycin C, actinomycin, bleomycin hydrochloride, and taxol. The drug can be bound to either the N-terminal of the A block or the C-terminal of the C block or can be bound to both the N-terminal of the A block and the C-terminal of the C block. However, when the drug is bound to both the N-terminal of the A block and the C-terminal of the C block, it is necessary not to inhibit the binding of the VEGF and the VEGF receptor.

The drug complex according to the present invention is typically used as a pharmaceutical composition. A pharmaceutical composition may contain, in addition to an effective amount of the VEFG-binding peptide-drug complex, auxiliary agents for pharmacologically acceptable formulations. The auxiliary agents may be, for example, an excipient, a binder, a disintegrant, a lubricant, a coating agent, a flavoring agent, and a solubilizer. The composition is provided in a form (dosage form) that can be applied orally or parenterally to animals including humans. The dosage form can be, for example, a tablet, a granule, a powder, a liquid, an injection, or a suppository.

A dose of the VEFG-binding peptide-drug complex is appropriately determined by a person skilled in the art according to gender, body weight, age, race, symptoms, and the like. A lower limit of the dose can be, for example, 0.001 µg/kg body weight, 0.01 µg/kg body weight, 0.1 µg/kg body weight, 0.001mg/kg body weight, 0.01mg/kg body weight, 0.05mg/kg body weight, and 0.1mg/kg body weight. Further, an upper limited of the dose can be, for example, 1000 mg/kg body weight, 100 mg/kg body weight, 10 mg/kg body weight, 5 mg/kg body weight, and 1 mg/kg body weight.

Next, the present invention is described in more detail based on the following examples. However, the present invention is not limited to the following examples.

### [Example 1]

### [Synthesis of VEGF-binding peptide Derivative]

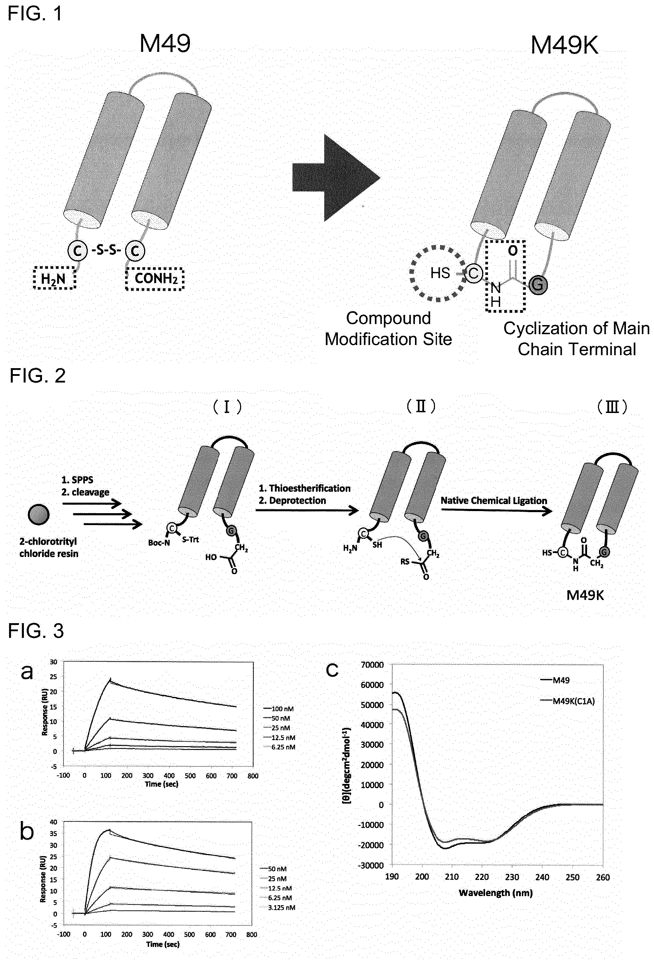

A linker for drug binding was introduced with respect to a cyclic VEGF target peptide (M49) stabilized by a disulfide bond, and a VEGF-binding peptide derivative (M49K), in which a main chain portion of a peptide was cyclized in order to stabilize the peptide, as synthesized (Fig. 1). Table 2 shows amino acid sequences of the peptide M49 and the peptide M49K. The sequence of the peptide M49 has an A block having an amino acid sequence shown in SEQ ID NO: 7, a B block having an amino acid sequence shown in SEQ ID NO: 6, and a C block having an amino acid sequence shown in SEQ ID NO: 13. As shown in Fig. 1, a cysteine that is an N-terminal amino acid and a cysteine that is a C-terminal amino acid are cyclized by an SS bond, and a carboxyl group of the C-terminal is aminated. The peptide M49K is cyclized by deleting one amino acid (alanine) from the A block, deleting a C-terminal cysteine of the C block, and binding four glycines as linkers.

**[Table 2]**

| | Amino Acid Sequence | SEQ ID NO. |
|---|---|---|
| M49 | CAAELAALEAELAALEGPWKGYPIPYGKLQFLIKKLKQLKVAC | 1 |
| M49K | CAELAALEAELAALEGPWKGYPIPYGKLQFLIKKLKQLKVAGGGG | 2 |

Fmoc-Gly-OH, DIEA, and DMF/DCM were added to a 2-chlorotrityl chloride resin to bind Fmoc-glycine, and thereafter, DIEA/MeOH/DCM was added to cap unreacted trityl groups. Using a capped Fmoc-Gly-trityl resin, a peptide having an amino acid sequence shown in SEQ ID NO: 2 was synthesized using a Fmoc solid-phase synthesis method. After the solid phase synthesis, resin removal was performed using DCM/TFE/AcOH (Fig. 2 (I)). BOP, DIEA, 3-mercaptopropionate, and chloroform were added to a residue obtained by the resin removal and the mixture was stirred at a room temperature to thioesterify a C-terminal. After completion of the reaction, TFA/H2O/phenol/TIS (88/5/5/2) was added to a residue obtained by removing a solvent, and the mixture was stirred at a room temperature to perform deprotection of a side chain protecting group of the peptide. After completion of the reaction, diethyl ether was added and a precipitated peptide was collected by centrifugal separation and was dried. The obtained crude peptide was dissolved in a 0.1% TFA aqueous solution and was purified using RP-HPLC (Fig. 2 (II)). Next, the C-terminal and the N-terminal were condensed by the following native chemical ligation reaction to synthesize a cyclized peptide M49K. The thioesterified M49 of Fig. 2 (II) was dissolved in water and was added dropwise to a reaction solution containing 200 mM Na2HPO4, 2 mM MPAA, and 20 mM TCEP·HCl. After completion of the reaction, freeze-drying was performed, and an obtained powder was dissolved in a 0.1% TFA solution and was purified using RP-HPLC (Fig. 2 (III)). With respect to the purified peptide M49K, purity was measured using RP-HPLC, and a molecular weight was confirmed using MALDI-TOF-MS (purity: 95%; [M+H]+: calculated value 4724.656 : measured value 4724.66).

Further, when the physical properties of the peptide M49K were measured, in order to prevent formation of a dimer via a thiol group, a peptide derivative M49K (C1A) in which a cysteine residue of the C-terminal of the M49K was substituted with an alanine residue in a desulfurization reaction was also synthesized. The obtained M49K was added to a reaction solution containing 400 mM Tris, 250 mM TCEP·HCl and 200 mM VA-044, and glutathione, and the mixture was allowed to react at a room temperature to obtain the M49K (C1A). A molecular weight of this peptide derivative was also confirmed using MALDI-TOF-MS, and it was confirmed that a desired peptide was synthesized.

Next, in order to investigate whether or not the peptide M49K (C1A) has the same physical properties as the peptide M49, using a surface plasmon resonance (SPR) method, a dissociation constant with respect to VEGF was measured, and information about a secondary structure was obtained based on a circular dichroism (CD) spectrum. The VEGF was immobilized to a sensor chip CM5 at 300 RU by amine coupling, and binding parameters were calculated from a sensorgram obtained by adding a peptide of each concentration using a 1:1 binding model. Further, the CD spectrum was measured at 20 °C in a 20 mM phosphate buffer solution (pH 7.0) having a peptide concentration of 20 µM. As shown in Fig. 3 and Table 3, the peptide M49 and the peptide derivative M49K (C1A), which is obtained by subjecting the peptide M49 to modification, have similar physical properties. It was concluded that the stabilized cyclic peptide M49K has the same physical properties as the peptide M49.

**[Table 3]**

| | *k*ₐ (1/Ms) | *k*_{d} (1/s) | *K*_{D} (nM) |
|---|---|---|---|
| M49 | (9.2 ± 1.9) × 10⁵ | (7.6 ± 0.1) × 10⁻⁴ | 0.87 ± 0.15 |
| M49K(C1A) | (4.5 ± 0.6) × 10⁵ | (2.0 ± 0.6) × 10⁻³ | 4.5 ± 1.1 |

### [Incorporation of Peptide Derivative M49K into Cell]

The peptide derivative M49K does not inhibit interaction between a VEGF and a VEGF receptor. Therefore, next, whether or not the M49K, together with a VEGF, was incorporated into HUVEC (human umbilical vein endothelial cells) by endocytosis via a VEGF receptor was investigated. HUVEC was seeded and cultured in a glass base dish. A VEGF fluorescently labeled with Alexa-488 and the M49K fluorescently labeled with Cy5 were added to the HUVEC in the dish, and after 6 hours, laser confocal microscopy observation was performed. As a result, as shown in Fig. 4, fluorescence of Alexa-488 and fluorescence of Cy5 were observed in the cells. Further, since these fluorescences were co-localized, it was found that the M49K was incorporated into cells together with the VEGF.

In addition, fluorescence intensities of the cells were measured using a flow cytometer. The HUVEC was seeded in a 96-well flat bottom plate, VEGF (2 µg/mL) fluorescently labeled with Alexa-488 and/or M49K (100 nM) fluorescently labeled with Cy5 were added, and the plate was placed in a 5% CO₂ incubator at 37 °C for 48 hours. Thereafter, a trypsin treatment was performed, and washing with PBS was performed, and thereafter, analysis using a flow cytometer was performed. As a result, for HUVEC that was not subjected to any treatment, the fluorescence intensities of Cy5 and Alexa488 were both low (Fig. 5A). When only VEGF-Alexa488 was added, a cell population was observed in a region where the fluorescence intensity of Alexa-488 was strong (Fig. 5B). When only M49K-Cy5 was added to the HUVEC, fluorescence intensity of a cell population was observed in the same area as the untreated HUVEC (Fig. 5C). Finally, when M49K-Cy5 and VEGF-Alexa488 were added at the same time, the cell population shifted to a region where the fluorescence intensities of Cy5 and Alexa488 were both high (Fig. 5D). That is, M49K was shown to be incorporated into the HUVEC only in the presence of VEGF.

### [Synthesis of Cyclic VEGF Target Peptide-Drug Complex]

Next, a drug was bound to the peptide derivative M49K, and a drug complex was synthesized. As the drug, CemCH2-SH, which is an analogue of cemadotin, which is a tubulin polymerization inhibitor, was used, and was bounded to a cysteine residue of the M49K via a disulfide bond. CemCH2-SH is a commonly known compound (Bemardes, G. J. L. et al. Angew. Chem. Int. Ed. 124, 965-968 (2012)). Due to the presence of a large amount of glutathione, an intracellular condition is likely to be reductive. Therefore, it can be imagined that, when the M49K-drug complex is incorporated into the cells, the disulfide bond is cut and the drug exhibits toxicity.

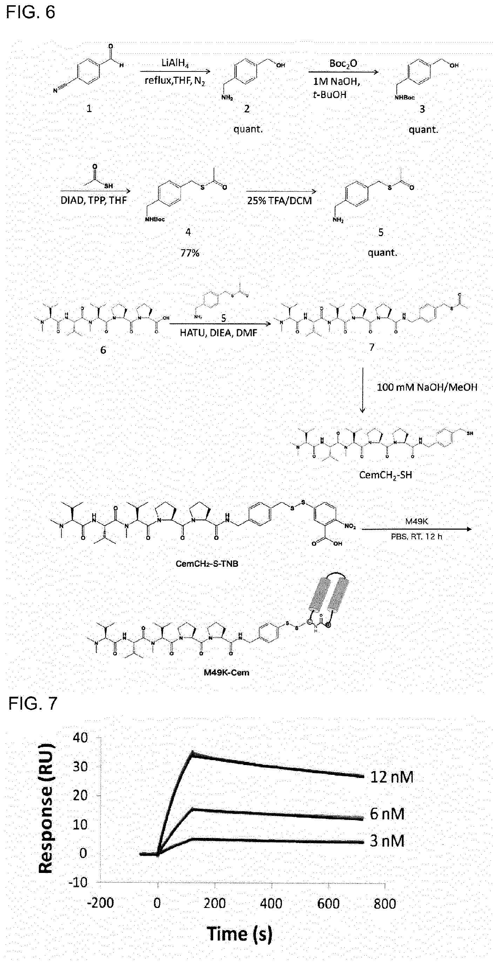

CemCH2-SH was synthesized according to the scheme of Fig. 6. First, 4-Cyanobenzaldehyde (1) was dissolved in anhydrous THF to which LiAlH4 was added, and the solution was allowed to react under reflux under a nitrogen atmosphere, and thereafter, NaOH was added to obtain (4-(aminomethyl) phenyl) methanol (2). An amino group of the compound 2 was protected with a Boc group, and thereafter, the compound was allowed to react with di-tert-butyl dicarbonate in tBuOH/NaOH. From the reaction solution, by extraction with a saturated aqueous sodium hydrogen carbonate solution and ethyl acetate, tert-butyl 4-(hydroxymethyl) benzylcarbamate (3) was obtained. Next, the compound 3 and triphenylphospine and thioacetic acid were dissolved in anhydrous THF, and diisopropyl azodicarboxylate was added, and the solution was allowed to react at 0 °C, and thereafter, diethyl ether was added to obtain an organic layer. By distilling off an organic solvent under a reduced pressure, S-4-(((tert-Butoxycarbonyl) amino) methyl) benzyl ethanethioate (4) was obtained. The compound 4 was dissolved in dichloromethane and a Boc group was deprotected by adding TFA, and S-4-(Aminomethyl) benzyl ethanethioate (5) was obtained.

Separately, N,N-dimethylvalyl-valyl-N-methylvalyl-prolyl-proline (6) was synthesized using an Fmoc solid phase synthesis method using 2-chlorotrityl chloride resin as a carrier. Fmoc-Pro-OH, DIEA, and DMF/DCM were added to a 2-chlorotrityl chloride resin to bind Fmoc-glycine, and thereafter, DIEA/MeOH/DCM was added to cap unreacted trityl groups. A capped Fmoc-Pro-trityl resin was used, and a solution prepared by dissolving an amino acid, HATU and DIEA in DMF was added to the resin to perform solid phase synthesis. Cleavage was performed using DCM/TFE/AcOH to obtain N,N-dimethylvalyl-valyl-N-methylvalyl-prolyl-proline (6). The compound 6, HATU and DIEA were dissolved in DMF and the solution stirred. The compound 5 was added thereto and the mixture was allowed to react while being stirred. DMF was distilled off under a reduced pressure, and, by performing dissolution in water/acetonitrile and purification using high performance liquid chromatography, CemCH2-SAc (7) was obtained. The compound (7) was dissolved in methanol, and thioester was converted to a thioether group in the presence of NaOH, and the reaction was stopped by adding a tris buffer solution and DTT, and CemCH2-SH was synthesized.

CemCH2-SH and 2,2'-Dithiobis (5-nitropyridine) were dissolved in THF and the solution was allowed to react at a room temperature for 2 hours and a solvent was distilled off. Thereby, CemCH2-S-TNB was obtained. The obtained CemCH2-S-TNB and M49K were dissolved in PBS and the solution was allowed to react at a room temperature. Thereby, M49K-Cem was synthesized. The synthesized M49K-Cem was purified using RP-HPLC. Purity was measured using RP-HPLC, and a purity of a purified M49K-Cem was 95%. Further, a molecular weight was confirmed using MALDI-TOF-MS, and a measured value of the molecular weight was m/z = 5407.076. A calculated value of [M+H]+ of the complex M49K-Cem was 5407.052, and it was confirmed that a target compound M49K-Cem was synthesized.

Subsequently, binding activity with respect to VEGF was measured using the SPR method in the same manner as the M49K (C1A) (Fig. 7). As a result, kₐ was 6.6 × 10⁵ (1/Ms), k_{d} was 3.9 × 10⁻⁴ (1/s), and the dissociation constant K_{D} was 0.6 nM. Therefore, it was found that a conjugate of CemCH2-SH to M49K does not affect binding to VEGF.

### [Example 2]

### [Test of Cell Growth Inhibition by M49K-Cem]

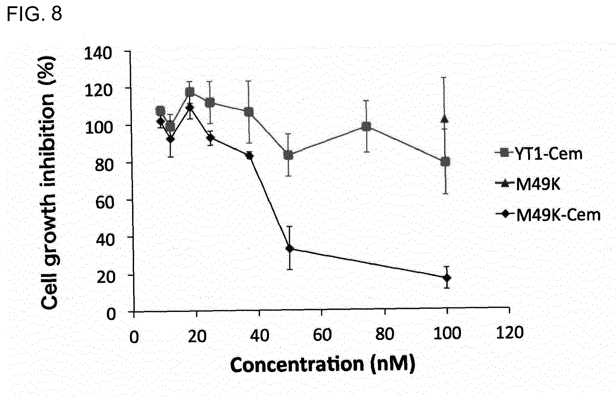

A growth inhibition test of HUVEC was performed using M49K-Cem. HUVEC (3000 cells / 100 µL / well) suspended in an EBM-2 culture medium was added to a 96-well flat bottom plate and was cultured overnight at 37 °C and 5% CO₂. Next, the culture medium was withdrawn, and a DMEM culture medium (0.2% FCS) containing 25 ng/mL VEGF and a sample of each concentration was added thereto. After culturing at 37 °C and 5% CO₂ for 24 hours, a cell growth degree was examined using a WST-1 assay (Ishiyama, M. et. al., Biol. Pharm. Bull. 19, 1518-1520 (1996)). As a result, as shown in Fig. 8, M49K-Cem inhibited cell growth in a concentration-dependent manner and an IC50 value with respect to HUVEC was 45 nM. On the other hand, as controls, M49K and YT1-Cem did not inhibit cell growth. YT1 is a helix-loop-helix peptide having an amino acid sequence shown in SEQ ID NO: 18 that does not bind to VEGF, and is obtained by causing YT1-Cys (CAELAALEAELAALEGGGGGGGKLAALKAKLAALKA-NH2) to react with CemCH2-S-TNB in PBS.

It is believed that M49K-Cem was incorporated into the cells and the bond between peptide and CemCH2-SH was cut. This is because CemCH2-SH does not exhibit toxicity unless separated from the peptide. Further, since YT1-Cem was not incorporated into the cells, it is presumed that the binding between the peptide and CemCH2-SH was not cut and cytotoxicity was not exerted.

### [Industrial Applicability]

According to the present invention, a new drug delivery system that allows a medicinal effect to be exerted in a cell expressing a VEGF receptor is provided.

## Claims

1. A VEGF-binding peptide-drug complex formed by binding a VEGF-binding peptide and a drug to each other, the VEGF-binding peptide having a helix-loop-helix structure including an A block that includes a peptide forming an α-helix structure and is positioned on an N-terminal side, a C block that includes a peptide forming an α-helix structure and is positioned on a C-terminal side, and a B block that includes a peptide linking the A block and the C block by a covalent bond, wherein
the drug directly or indirectly binds to an N-terminal amino acid and/or a C-terminal amino acid of the VEGF-binding peptide and is incorporated into a VEGF receptor-expressing cell by endocytosis of a VEGF receptor.

2. The VEGF-binding peptide-drug complex according to claim 1, wherein the drug is bound to the N-terminal amino acid or the C-terminal amino acid of the VEGF-binding peptide in a binding mode that allows the binding to be cut by an intracellular enzyme.

3. The VEGF-binding peptide-drug complex according to any one of claims 1 and 2, wherein the drug is directly bound to a condensable functional group of the N-terminal amino acid or the C-terminal amino acid of the VEGF-binding peptide.

4. The VEGF-binding peptide-drug complex according to any one of claims 1 and 2, wherein the condensable functional group is any one of a thiol group, an amino group, a hydroxyl group, a carboxyl group, and an aldehyde group.

5. The VEGF-binding peptide-drug complex according to any one of claims 1 - 4, wherein the N-terminal amino acid of the VEGF-binding peptide and the C-terminal amino acid of the VEGF-binding peptide are directly or indirectly bound to each other.

6. The VEGF-binding peptide-drug complex according to claim 5, wherein the N-terminal amino acid of the A block and the C-terminal amino acid of the B block are bound to each other by a linker including of 1 - several amino acids.

7. The VEGF-binding peptide-drug complex according to any one of claims 1 - 6, wherein the B block is a peptide that includes an amino acid sequence described in any one of SEQ ID NOs: 3 - 6.

8. The VEGF-binding peptide-drug complex according to claim 7, wherein
the A block is a peptide that includes an amino acid sequence described in SEQ ID NO: 7, and
the C block is a peptide that includes an amino acid sequence described in SEQ ID NO: 8.

9. The VEGF-binding peptide-drug complex according to claim 7, wherein
the A block is a peptide that includes an amino acid sequence described in SEQ ID NO: 7, and
the C block is a peptide that includes an amino acid sequence described in SEQ ID NO: 9 (KLXXLKXKLXXLKXAC: where X is an amino acid other than threonine, alanine and proline).

10. The VEGF-binding peptide-drug complex according to claim 7, wherein
the A block is a peptide that includes an amino acid sequence described in SEQ ID NO: 7, and
the C block is a peptide that includes an amino acid sequence described in any one of SEQ ID NOs: 10 - 13.

11. A VEGF-binding peptide having a helix-loop-helix structure including an A block that includes a peptide forming an α-helix structure and is positioned on an N-terminal side, a C block that includes a peptide forming an α-helix structure and is positioned on a C-terminal side, and a B block that includes a peptide linking the A block and the C block by a covalent bond, wherein
an N-terminal amino acid and/or a C-terminal amino acid of the VEGF-binding peptide have/has a free condensable functional group to form a non-cyclic VEGF-binding peptide (however, a VEGF-binding peptide in which both an N-terminal amino acid and a C-terminal amino acid are cysteines is excluded).

12. A VEGF-binding peptide having a helix-loop-helix structure including an A block that includes a peptide forming an α-helix structure and is positioned on an N-terminal side, a C block that includes a peptide forming an α-helix structure and is positioned on a C-terminal side, and a B block that includes a peptide linking the A block and the C block by a covalent bond, wherein
an N-terminal amino acid or a C-terminal amino acid of the VEGF-binding peptide has a free condensable functional group, and the N-terminal amino acid of the VEGF-binding peptide and the C-terminal amino acid of the VEGF-binding peptide are directly or indirectly bound to each other to form a cyclic VEGF-binding peptide (however, a VEGF-binding peptide in which an N-terminal amino acid and a C-terminal amino acid are directly bound to each other by a SS bond is excluded).

13. The VEGF-binding peptide according to claim 12, wherein the N-terminal amino acid of the VEGF-binding peptide and the C-terminal amino acid of the VEGF-binding peptide are bound to each other by an acid amide bond.

14. The VEGF-binding peptide according to claim 12, wherein an N-terminal amino acid of the A block and a C-terminal amino acid of the C block are bound to each other by a linker including of 1 - several amino acids.

15. The VEGF-binding peptide according to any one of claims 11 - 14, wherein the condensable functional group is any one of a thiol group, a hydroxyl group, an amino group, a carboxyl group, and an aldehyde group.


SEQUENCE LISTING

<110>  Osaka Prefecture University Public Corporation

<120>  Drug conjugate

<130>  15PCT11703

<160>  18

<170>  PatentIn version 3.5

<210>  1
<211>  43
<212>  PRT
<213>  Artificial Sequence

<220>
<223>  VEGF-Binding Peptide M49

<400>  1

Cys Ala Ala Glu Leu Ala Ala Leu Glu Ala Glu Leu Ala Ala Leu Glu
1               5                   10                  15

Gly Pro Trp Lys Gly Tyr Pro Ile Pro Tyr Gly Lys Leu Gln Phe Leu
            20                  25                  30

Ile Lys Lys Leu Lys Gln Leu Lys Val Ala Cys
        35                  40

<210>  2
<211>  45
<212>  PRT
<213>  Artificial Sequence

<220>
<223>  VEGF-Binding Peptide M49K

<400>  2

Cys Ala Glu Leu Ala Ala Leu Glu Ala Glu Leu Ala Ala Leu Glu Gly
1               5                   10                  15

Pro Trp Lys Gly Tyr Pro Ile Pro Tyr Gly Lys Leu Gln Phe Leu Ile
            20                  25                  30

Lys Lys Leu Lys Gln Leu Lys Val Ala Gly Gly Gly Gly
        35                  40                  45

<210>  3
<211>  11
<212>  PRT
<213>  Artificial Sequence

<220>
<223>  B-Block of Clone 36

<400>  3

Gly Thr Tyr Arg Ala Ser Thr Trp Trp Trp Gly
1               5                   10


<210>  4
<211>  11
<212>  PRT
<213>  Artificial Sequence

<220>
<223>  B-Block of Clone 41

<400>  4

Gly Pro Asp Leu Met Val Trp Trp Gly Trp Asp
1               5                   10


<210>  5
<211>  11
<212>  PRT
<213>  Artificial Sequence

<220>
<223>  B-Block of Clone 42

<400>  5

Gly Asn Ser Asp Tyr Pro Trp Ile Gly Trp Gly
1               5                   10


<210>  6
<211>  11
<212>  PRT
<213>  Artificial Sequence

<220>
<223>  B-Block of Clone 49

<400>  6

Gly Pro Trp Lys Gly Tyr Pro Ile Pro Tyr Gly
1               5                   10


<210>  7
<211>  16
<212>  PRT
<213>  Artificial Sequence

<220>
<223>  A block

<400>  7

Cys Ala Ala Glu Leu Ala Ala Leu Glu Ala Glu Leu Ala Ala Leu Glu
1               5                   10                  15

<210>  8
<211>  16
<212>  PRT
<213>  Artificial Sequence

<220>
<223>  A block

<400>  8

Lys Leu Ala Ala Leu Lys Ala Lys Leu Ala Ala Leu Lys Ala Ala Cys
1               5                   10                  15


<210>  9
<211>  16
<212>  PRT
<213>  Artificial Sequence

<220>
<223>  Replaced c-Block



<220>
<221>  MISC_FEATURE
<222>  (3)..(3)
<223>  Xaa is any amino acid except Threonine, Alanine and Proline

<220>
<221>  MISC_FEATURE
<222>  (4)..(4)
<223>  Xaa is any amino acid except Threonine, Alanine and Proline

<220>
<221>  MISC_FEATURE
<222>  (7)..(7)
<223>  Xaa is any amino acid except Threonine, Alanine and Proline

<220>
<221>  MISC_FEATURE
<222>  (10)..(10)
<223>  Xaa is any amino acid except Threonine, Alanine and Proline

<220>
<221>  MISC_FEATURE
<222>  (11)..(11)
<223>  Xaa is any amino acid except Threonine, Alanine and Proline

<220>
<221>  MISC_FEATURE
<222>  (14)..(14)
<223>  Xaa is any amino acid except Threonine, Alanine and Proline

<400>  9

Lys Leu Xaa Xaa Leu Lys Xaa Lys Leu Xaa Xaa Leu Lys Xaa Ala Cys
1               5                   10                  15


<210>  10
<211>  16
<212>  PRT
<213>  Artificial Sequence

<220>
<223>  C-Block of Clone 36

<400>  10

Lys Leu Phe Gln Leu Lys Asn Lys Leu His Gln Leu Lys Tyr Ala Cys
1               5                   10                  15


<210>  11
<211>  16
<212>  PRT
<213>  Artificial Sequence

<220>
<223>  C-Block of Clone 41

<400>  11

Lys Leu Asn Gln Leu Lys His Lys Leu Asp His Leu Lys Val Ala Cys
1               5                   10                  15


<210>  12
<211>  16
<212>  PRT
<213>  Artificial Sequence

<220>
<223>  C-Block of Clone 42

<400>  12

Lys Leu Gly Glu Leu Lys Gln Lys Leu Leu Lys Leu Lys Asn Ala Cys
1               5                   10                  15


<210>  13
<211>  16
<212>  PRT
<213>  Artificial Sequence

<220>
<223>  C-Block of Clone 49

<400>  13

Lys Leu Gln Phe Leu Ile Lys Lys Leu Lys Gln Leu Lys Val Ala Cys
1               5                   10                  15


<210>  14
<211>  43
<212>  PRT
<213>  Artificial Sequence

<220>
<223>  VEGF-Binding Peptide No.36

<400>  14

Cys Ala Ala Glu Leu Ala Ala Leu Glu Ala Glu Leu Ala Ala Leu Glu

1               5                   10                  15

Gly Thr Tyr Arg Ala Ser Thr Trp Trp Trp Gly Lys Leu Phe Gln Leu
            20                  25                  30

Lys Asn Lys Leu His Gln Leu Lys Tyr Ala Cys
        35                  40

<210>  15
<211>  43
<212>  PRT
<213>  Artificial Sequence

<220>
<223>  VEGF-Binding Peptide No.41

<400>  15

Cys Ala Ala Glu Leu Ala Ala Leu Glu Ala Glu Leu Ala Ala Leu Glu
1               5                   10                  15

Gly Pro Asp Leu Met Val Trp Trp Gly Trp Asp Lys Leu Asn Gln Leu
            20                  25                  30

Lys His Lys Leu Asp His Leu Lys Val Ala Cys
        35                  40

<210>  16
<211>  43
<212>  PRT
<213>  Artificial Sequence

<220>
<223>  VEGF-Binding Peptide No.42

<400>  16

Cys Ala Ala Glu Leu Ala Ala Leu Glu Ala Glu Leu Ala Ala Leu Glu
1               5                   10                  15

Gly Asn Ser Asp Tyr Pro Trp Ile Gly Trp Gly Lys Leu Gly Glu Leu
            20                  25                  30

Lys Gln Lys Leu Leu Lys Leu Lys Asn Ala Cys
        35                  40

<210>  17
<211>  43
<212>  PRT
<213>  Artificial Sequence

<220>
<223>  VEGF-Binding Peptide No.49

<400>  17

Cys Ala Ala Glu Leu Ala Ala Leu Glu Ala Glu Leu Ala Ala Leu Glu
1               5                   10                  15

Gly Pro Trp Lys Gly Tyr Pro Ile Pro Tyr Gly Lys Leu Gln Phe Leu
            20                  25                  30

Ile Lys Lys Leu Lys Gln Leu Lys Val Ala Cys
        35                  40

<210>  18
<211>  36
<212>  PRT
<213>  Artificial Sequence

<220>
<223>  VEGF Non Binding Peptide YT1

<400>  18

Cys Ala Glu Leu Ala Ala Leu Glu Ala Glu Leu Ala Ala Leu Glu Gly
1               5                   10                  15

Gly Gly Gly Gly Gly Gly Lys Leu Ala Ala Leu Lys Ala Lys Leu Ala
            20                  25                  30

Ala Leu Lys Ala
        35